Europäisches Patentamt

European Patent Office

Office européen des brevets

(11) Veröffentlichungsnummer: **0 319 676**

**A2**

# EUROPÄISCHE PATENTANMELDUNG

(21) Anmeldenummer: **88117116.9**

(22) Anmeldetag: **14.10.88**

(51) Int. Cl.⁴: **A61K 31/73**

---

Die Bezeichnung der Erfindung wurde geändert (Richtlinien für die Prüfung im EPA, A-III, 7.3).

(30) Priorität: **15.10.87 DE 3734962**

(43) Veröffentlichungstag der Anmeldung:
**14.06.89 Patentblatt 89/24**

(84) Benannte Vertragsstaaten:
**AT BE CH DE ES FR GB GR IT LI LU NL SE**

(71) Anmelder: **LUITPOLD-WERK**
**Chemisch-pharmazeutische Fabrik GmbH & Co.**
**Postfach 70 12 09**
**D-8000 München 70(DE)**

(72) Erfinder: **Panse, Peter, Dr. rer. nat.**
**Dipl.-Chem.**
**Scheinerstrasse 8**
**D-8000 München 80(DE)**

(74) Vertreter: **Zumstein, Fritz, Dr. et al**
**Zumstein & Klingseisen Patentanwälte**
**Bräuhausstrasse 4**
**D-8000 München 2(DE)**

---

(54) **Chondroitinpolysulfat enthaltende Zusammensetzungen zur Behandlung von durch Retroviren verursachten immundefizienten Zuständen.**

(57) Die Erfindung betrifft die Verwendung von Chondroitinpolysulfat und dieses enthaltenden pharmazeutischen Zusammensetzungen zur vorbeugenden, lindernden oder heilenden Behandlung von Infektionen und nachfolgenden pathologischen Zuständen mit Immunschwäche, die durch Immunschwäche erzeugende Retroviren oder deren Enzyme verursacht werden.

EP 0 319 676 A2

# Verwendung von Chondroitinpolysulfat und dieses enthaltenden pharmazeutischen Zusammensetzungen zur Behandlung von Infektionen und pathologischen Zuständen mit Immundefizienz, die durch Immunschwäche erzeugende Retroviren verursacht sind.

Die Erfindung betrifft die Verwendung von Chondroitinpolysulfat (CPS) zur Prophylaxe, Linderung oder Behandlung von pathologischen Zuständen mit Immundefizienz, die durch Retroviren und ihre Enzyme verursacht sind.

Bei verschiedenen Retroviren, wie dem Friend Murine Leukaemia Virus (FMLV) ,dem Feline Immunodeficiency Virus (FIV), dem Simian Immunodeficiency Virus (SIV) und vor allem dem die Krankheit Acquired Immunodeficiency Syndrom (AIDS) verursachenden Human Immunodeficiency Virus (HIV) handelt es sich um Viren, welche die Wirtszellen (insbesondere die T-Zellen) befallen und diese durch die RNS abhängige DNS-Polymerase [d.i. Reverse Transkriptase (RT)] derart verändern, daß die Immunantwort auf Antigene unterdrückt wird.

Antagonisten gegen Retroviren und gegen die von ihnen ausgelösten Effekte werden benötigt, um pathologische Zustände und Krankheiten, an denen diese Viren beteiligt sind, zu behandeln. Indikationen für z.B. Antagonisten von HIV sind AIDS und AIDS Related Complex (ARC), sowie auch symptomlose Infektionen mit den genannten Viren.

Es sind bereits Substanzen mit antagonisticher Wirkung gegen Retroviren bekannt geworden, zum Beispiel:

a) Substanzen, deren Struktur Analogien zu den Purinbasen der RNS bzw. DNS aufweisen, wie zum Beispiel Azido-thymidin, 3-Azido-dideoxiguanosin, 3-Fluorcytosin;

b) Substanzen, die in der Zusammensetzung den Hüllenbestandteilen der Retroviren nahekommen und welche die für Viren geeigneten Akzeptorstellen der potentiellen Wirtszellen belegen, zum Beispiel Peptid T;

oder solche, die gegen reserve Transkriptase wirksam sind:

c) Substanzen mit anorganischen negativ geladenen Gruppen, wie: Suramin (H. Mitsuya et al., Science 226, 172-174 (1984) ; Phosphonoformiat [B. Öberg, Pharmac. Ther. 19, 387-415 (1983)], sowie Polysaccharidpolyschwefelsäureester wie z.B. die in der DE OS 36 61 136 beschriebenen Pentosanpolysulfat, Dextransulfate und Chondroitinsulfat.

Es ist ferner bekannt, daß polyanionische Polysaccharide virushemmend wirken. A. Vaheri beschrieb dies u.a. für Heparin (Acta patholog. et microbiologica scand. Suppl. 171, SS. 1-98 (1964).

H. Voß faßte in der DE-OS 312 4384 die bis dahin erschienenen Publikationen zur Virushemmung durch polyanionische Verbindungen zusammen. So wurde die Wirkung von Heparin, Chondroitinsulfat und Keratansulfat auf Herpes simplex Virus (HSV) [A. Vaheri (1964), siehe oben, K.K. Takemoto und P. Fabisch, Proc.Soc.exp.Biol. (N.Y.) 116, 140-144 (1964) ,A.J.Nahmias, S. Kibrick und P. Bernfeld, Proc.Soc.Biol.Med. 115, 993996 (1964)] beschrieben.

Ferner beschrieben H. Voß, M. Mündler und G. Plötze, Zbl. Bakt. I.O. 192, 137-157 (1964) sowie H. Voß, Zbl.Bakt.I.O. 198, 211-213 (1965) die Wirkung von Agarmucopolysaccharid, Heparin, Chondroitinsulfat und Dextransulfaten auf Vertreter der Picornavirus-Gruppe, Polyomyelitis- und ECHO-(Entero-Cytopathogenic Human Orphan) Viren.

In anderen Untersuchungen wurden die Wirkung von Chondroitinsulfat, Heparin und Chondroitinpolysulfat auf den Gelbfiebervirus-Impfstamm 17 D [H. Voß,J. Haase,H. Kauffman,G. Plötze und A. Ortmann, Zbl.Bakt.I.O. 209, 447-464 (1969),H. Voß, K.H. Sensch u.P. Panse, Zbl.Bakt.Hyg.,I.Abt.Orig. A 229, 1-36 (1974), sowie zusätzlich auf Herpes simplex Viren (HSV) und Influenza-Viren ( H. Voß, K.H. Sensch und P. Panse, Zbl.Bakt.Hyg., I.Abt.Orig. A 229,1-36( 1974)] festgestellt. Auch die lokale Anwendung von CPS bei Patienten mit Gürtelrose wurde beschrieben [H. Voß, H.D. Pohle und C. Museteanu, Zbl.Bakt.Hyg., I.Abt.Orig. A 237,1-34( 1977)], sowie H. Voß, J.of Pharmacotherapy 3, 84-87 (1980).

In der DE-OS 312 4384 beschreibt Voß die virushemmende Wirkung von Dermatanpolysulfat (DPS) gegenüber HSV und Gelbfieber-Virus 17 D.

In der DE-OS 2051 745 wird von U. Kuhlmann die Wirkung von chemisch modifizierten, insbesondere methylierten Ribonukleinsäuren und Desoxyribonukleinsäuren beschrieben. Untersucht wurde das Myxovirus Parainfluenzae I.

Die Einwirkung von Polyanionen auf die Reverse Transcriptase (RT) des Avian Myeloblastosis Virus (AMV) wurde von D. Schaffrath, H.W. Stuhlsatz und H. Greiling, Hoppe Seyler's Z.Physiol.Chem. 357, 499-508 (1976) beschrieben. Verbindungen wie z.B. das Glykosaminoglykanpolysulfat Chondroitinpolysulfat (CPS), Chondroitin-4-sulfat und Chondroitin hemmten die aus dem Vogel Tumor-Virus AMV gewonnene Reverse Transcriptase. CPS zeigte dabei eine deutliche Enzymhemmung bei 10 µMol/l.

In der oben genannten DE-OS 36 61 136 wird ebenfalls die Hemmung dieses Enzyms AMV-Reverse Transcriptase (AMV-RT) durch Polyanionen beschrieben. Es wurde dort der Schluß gezogen, die mit AMV-RT gewonnenen Ergebnisse auch auf die Hemmwirkung bei Säuger-Retroviren übertragen zu können.

Eine Wirkung von CPS gegen die Immunschwäche auslösenden Viren ist bisher trotz der intensiven Suche nach wirksamen Inhibitoren nicht bekannt geworden.

Es wurde nun überraschend festgestellt, daß CPS eine ausgeprägt starke Hemmwirkung auf die Einwirkung von HIV auf Kulturen von menschlichen T-Zellen hat. Die starke Hemmwirkung von CPS gegenüber HIV beginnt bereits bei Konzentrationen unterhalb von 0,1 µg/ml.

Ein unerwarteter Vorteil bei der starken virushemmenden Wirkung ist die geringe Toxizität von CPS gegenüber den $MT_4$-Zellen, bis hinauf zu 12500 µg/ml. Die bekannte Wirksubstanz Azidothymidin weist in vitro im Bereich von 40 bis 200 µg/ml bereits starke Zelltoxizität auf, seine klinischen Nebenwirkungen sind bekannt. Eine Substanz mit geringer Toxizität bei hoher Wirksamkeit ist ein wünschenswertes Ziel. Das Zusammentreffen beider Effekte ist bei den bisher untersuchten Wirkstoffen gegen AIDS vergeblich gesucht worden; CPS ermöglicht nun bei der Anwendung eine LangzeitProphylaxe bzw. -Therapie, zumal die für diese Substanz erzielten Serumspiegel beim Menschen nach Dosierung von ca. 1mg/kg innenhalb von 48 h noch im Bereich von 0,1 µg/ml liegen [W. Müller, P. Panse, S. Brand und A. Staubli, Z.Rheumatol.42, 355-361 (1983)].

Nach der bisherigen Auffassung (DE-OS 36 61 136) soll die gegenüber AMV-RT gemessene Hemmwirkung einer Substanz auch auf Säuger-Retroviren-RT übertragbar sein. Wie jedoch Versuche der Anmelderin ergeben, ist eine Voraussage der Hemmung der Reversen Transcriptasen verschiedener immunschwächeerzeugender Retroviren durch Wirkstoffe aufgrund ihrer Hemmung von AMV-RT nicht möglich.

Gegenstand der Erfindung ist die Verwendung von Chondroitinpolysulfat und dieses enthaltenden pharmazeutischen Zusammensetzungen zur vorbeugenden, lindernden und/oder heilenden Behandlung von Infektionen und nachfolgenden pathologischen, z.T. symptomlosen Zuständen mit Immunschwäche, die durch Immunschwäche erzeugende Retroviren oder deren Enzyme verursacht werden, bei Säugern. Insbesondere betrifft die Erfindung die vorstehende Verwendung zur vorbeugenden, lindernden oder heilenden Behandlung von durch das Human Immuno Deficiency Virus (HIV) verursachten Infektionen und nachfolgenden pathologischen Zuständen, vor allem von AIDS (Acquired Immuno Deficiency Syndrom) und ARC (AIDS Related Complex).

CPS ist eine bekannte Substanz. Zur erfindungsgemäßen Verwendung ist jedes, je nach Applikationsart verträgliche CPS geeignet. Herstellverfahren für zu verwendendes CPS sind z.B. in der DE-PS 870094, US-PS 3454560 und DE-PS 3118588 beschrieben.

Die bestimmungsgemäße Verwendung von CPS kann bei warmblütigen Tieren und Beim Menschen unter anderem topisch, parenteral durch s.c., i.m. oder i.v. Injektion, durch Schleimhautapplikation, als Vaginalstylos, mittels Kondom, Gel, Salbe oder durch Inhalation eines Aerosols erfolgen.

In den Darreichungsformen, die oben nur beispielhaft aufgeführt sind, ist CPS entweder ohne oder zusammen mit üblichen pharmazeutischen Hilfsstoffen enthalten. Spezielle Applikations formen sind beispielsweise,ohne die Erfindung darauf zu beschränken, Kapseln, Pulver, Lösungen, Injektionslösungen, Infusionslösungen, Suspensionen,Aerosole, Implantate, Salben, Emulsionen, Sirupe, Suppositorien usw.

Wirksame Einzeldosen von CPS liegen bei systemischer Anwendung beispielsweise zwischen 1 und 500 mg, vorzugsweise zwischen 25 und 100 mg, bei oraler Anwendung und lokaler Applikation (Haut, Schleimhaut oder Gewebe) beispielsweise zwischen 1 und 500 mg, vorzugsweise zwischen 10 und 100 mg. Die systemisch anzuwendenden Einzeldosen können täglich bis maximal 5 mal, vorzugsweise bis 2 mal, die oral oder lokal applizierten Einzeldosen bis 10 mal, vorzugsweise bis 2 mal gegeben werden. Die systemisch zu gebenden Zubereitungen enthalten CPS in Konzentrationen von beispielsweise zwischen 0,01 und 25 %,vorzugsweise zwischen 2,5 und 10 %. Die oral zu applizierenden Zubereitungen enthalten CPS in Konzentrationen von beispielsweise zwischen 0,02 und 100 %, vorzugsweise zwischen 2 und 80 %. Die lokal zu applizierenden Zubereitungen enthalten CPS in Konzentrationen von beispielsweise zwischen 0,01 und 100 %, vorzugsweise zwischen 1 und 90 %.

In vitro Versuche

Beispiel 1 Wirksamkeit und Toxizität im Zellkulturversuch

Die Wirksamkeit von CPS, hergestellt nach DE-PS 3118588, wurde in einem biologischen System festgestellt, bei welchem der aus Patienten mit AIDS und ARC isolierte HIV auf Humanzellinien gezüchtet wurde. Die in vitro Pathogenität des so gezüchteten HIV wurde unter Verwendung von M $T_4$-Zellen, einer $T_4$-Zellinie, für welche HIV cytopathisch ist, bestimmt. Die $MT_4$-Zellinie ist beschrie-

ben in Science, Vol. 229 (09. August 1985), Seiten 563-566. Der cytopathische Effekt wurde dabei durch Messung des Thymidineinbaus in die Zelle verfolgt. Wurde CPS in unterschiedlichen Konzentrationen den mit HIV inokulierten MT$_4$-Zellen zugesetzt, so gelang es bereits mit geringen Konzentrationen an CPS (unter 0,1 µg/ml), den durch das Virus hervorgerufenen cytopathischen Effekt zu verhüten.

Im gleichen System erfolgte auch die Toxizitätsbestimmung. Hierbei wurden MT$_4$-Zellen dem CPS in steigenden Konzentrationen in Abwesenheit von HIV ausgesetzt und anhand des Einbaus von radioaktivem Thymidin die Stoffwechselleistung der Zellen bzw. anhand deren Nachlassen die Toxizität des zugesetzten CPS bestimmt. Aus der so bestimmten Konzentration für die Erzielung einer antiviralen Wirksamkeit im Vergleich zu der gerade noch untoxischen Konzentration an Wirkstoff, wird im allgemeinen ein in vitro therapeutischer Index bestimmt. Dieser therapeutische Index ist für CPS bei der verwendeten humanen Zellinie größer als $1.25 \times 10^5$, da in der durchgeführten Versuchsreihe bei den höchsten Konzentrationen an CPS noch keine toxische Wirkung zu beobachten war.

Die Versuche wurden so durchgeführt, daß Proben des Überstandes der das Virus erzeugenden Zellinie in schrittweiser Verdünnung in Mikrotiterplatten eingebracht wurden, welche die Zellinie MT$_4$ enthalten. Gleichzeitig wurde in die Mikrotiterplatten radioaktiv markiertes Thymidin gegeben und der Einbau dieser Substanz, die der Lebenstätigkeit dieser Zellen parallel läuft, gemessen. Unter dem Einfluß zytopathogener Mengen an Virus enthaltender Flüssigkeit sinkt der Einbau an radioaktiv markiertem Thymidin entsprechend. Dies wird auch bei toxischen Konzentration anderer Wirkstoffe beobachtet.

Er zeigte sich, daß bei einer Verdünnung des Kulturüberstandes von 1:100 noch eine maximale Pathogenität des HIV gegenüber den MT$_4$-Zellen vorliegt. Die Untersuchung der Wirksamkeit und die Prüfung der Toxizität von CPS erfolgte bei dieser Virusverdünnung, indem eine vorgemischte Lösung von Virusverdünnung und des CPS der Zellinienprobe zugesetzt und wiederum der Thymidineinbau bestimmt wurde.

Selbst bei CPS-Konzentrationen unterhalb von 0.1 µg/ml sind die Zellen noch völlig gegen den pathogenen Effekt einer 1:100 Verdünnung von HIV-Stammflüssigkeit geschützt. Bis hinauf zu Konzentrationen von 12500 µg/ml weist der Wirkstoff keinerlei Toxizität auf. Dieses Ausbleiben der Toxizität ist für ein Langzeittherapeutikum, wie es für eine AIDS-Behandlung notwendig ist, äußerst bedeutungsvoll.

In vivo Versuche

Beispiel 2 Pharmakokinetische Versuche

Bei je drei 5 bis 7 kg schweren Rhesusaffen wurden intramuskulär (i.m.) isotone wässrige Lösungen von 5 bzw. 25 mg CPS pro Tier injiziert. Das CPS war dazu über die gesammte Molekülkette mit Tritium radioaktiv markiert worden. Die Tiere zeigten keinerlei Anzeichen von toxischer Einwirkung.

Serumproben wurden zu verschiedenen Zeitpunkten über einen Tag hin gewonnen, der Harn wurde in zwei Fraktionen von 0 bis 4 und von 4 bis 24 Stunden gesammelt. Alle Proben wurden radiometrisch gemessen. Die qualitative Erfassung von i.m. beim Menschen appliziertem CPS ist bei W. Müller, P. Panse, S. Brand und A. Staubli, Z. Rheumatol. 42, 355-361 (1983) beschrieben; es ergibt sich eine relativ gute Wiederfindung des Ausgangmoleküls.

Der Serumspiegel erreicht innerhalb kurzer Zeit sein Maximum und liegt nach einem Tag bei beiden Dosen noch oberhalb von 0,1 µg/ml.

Die Ausscheidungen im Harn betrugen innerhalb von 24 Stunden 52 % bzw. 50 % der applizierten Menge.

Pharmazeutische Zusammensetzungen

Die Herstellung von CPS-Zubereitungen zur Prophylaxe und Behandlung kann analog derjenigen der bekannten CPS- Zubereitungen erfolgen. Die Dosierung von CPS richtet sich nach dem vorher gesagten.

Beispiel 3

Ampullenlösung

Die Lösung setzt sich aus folgenden Bestandteilen zusammen:

| | |
|---|---|
| CPS | 50,0 g |
| Natriumchlorid | 5,0 g |
| Aqua ad iniectabilia | ad. 1000,0 g |

**Ansprüche**

1. Verwendung von Chondroitinpolysulfat zur Herstellung eines Arzneimittels zur vorbeugenden, lindernden oder heilenden Behandlung von Infektionen und nachfolgenden pathologischen Zuständen mit Immunschwäche, die durch Immunschwäche erzeugende Retroviren oder deren Enzyme verursacht werden.

2. Verwendung gemäß Anspruch 1 zur Herstellung eines Arzneimittels zur vorbeugenden, lindernden oder heilenden Behandlung von durch das Human Immunodeficiency Virus (HIV) verursachten Infektionen und nachfolgenden pathologischen Zuständen.

3. Verwendung gemäß Anspruch 1 und 2 zur Herstellung eines Arzneimittels zur vorbeugenden, lindernden oder heilenden Behandlung von AIDS (Acquired Immuno Deficiency Syndrom) und ARC (AIDS Related Complex).